(19) Europäisches Patentamt
European Patent Office
Office européen des brevets

(11) Numéro de publication: **0 356 333 A1**

(12) # DEMANDE DE BREVET EUROPEEN

(21) Numéro de dépôt: **89402322.5**

(22) Date de dépôt: **23.08.89**

(51) Int. Cl.[5]: **C 07 D 285/135**
C 07 D 417/12, A 61 K 31/41,
A 61 K 31/435

(30) Priorité: **25.08.88 FR 8811226**

(43) Date de publication de la demande:
**28.02.90 Bulletin 90/09**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Brodin, Roger**
**10, Rue de la Goelette Mas de Neuville**
**F-34100 Montpellier (FR)**

**Olliero, Dominique**
**12, Allée Jeanne Bourgeois Hameau de l'Aiguelongue**
**F-34100 Montpellier (FR)**

**Worms, Paul**
**10, Rue Devois**
**F-34980 Saint-Gely-Du-Fesc (FR)**

(74) Mandataire: **Gillard, Marie-Louise et al**
**Cabinet Beau de Loménie 55, Rue d'Amsterdam**
**F-75008 Paris (FR)**

Claims for the following Contracting States: ES + GR.

(54) **Dérivés du thiadiazole-1,3,4, leur procédé d'obtention et compositions pharmaceutiques en contenant.**

(57) La présente invention a pour objet des dérivés du thiadiazole de formule :

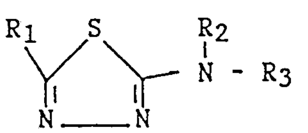

(I)

dans laquelle:
- $R_1$ représente un groupe phényle non substitué ou substitué 1 à 3 fois par un atome d'halogène, de préférence le chlore ou le fluor, par un groupe alkyle en $C_1$-$C_4$, de préférence le groupe méthyle, par un groupe alcoxy en $C_1$-$C_4$, de préférence le groupe méthoxy, par un groupe hydroxy, par un groupe trifluorométhyle ou un groupe phényle substitué à la fois par 1 à 3 atomes d'halogène et par 1 ou 2 groupements méthyle ;
- $R_2$ représente l'hydrogène ou un groupe alkyle en $C_1$-$C_4$
- $R_3$ représente un groupe alkylamino ou un hétérocycle.

Application : Composés ayant des propriétés antihistaminiques et anticholinergiques.

EP 0 356 333 A1

## Description

**Dérivés du thiadiazole-1,3,4, leur procédé d'obtention et compositions pharmaceutiques en contenant.**

La présente invention concerne de nouveaux dérivés du thiadiazole, leur procédé de préparation et leur application en thérapeutique.

Certains dérivés du thiadiazole sont connus dans l'art antérieur. la demande de brevet européen 183 577 décrit des dérivés de la (morpholino-2 éthylamino)-2 thiadiazole de formule :

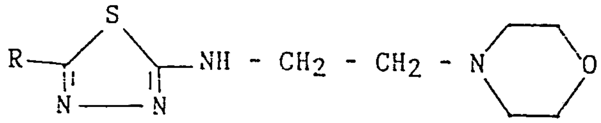

dans laquelle R représente un groupe alkyle, cycloalkyle, biphényle, naphtyle ou phényle (éventuellement substitué).

Ces composés présentent des propriétés antidépressives et dopaminomimétiques.

Par ailleurs, un article du Journal of Pharmaceutical Sciences, 1975, 64 n° 7, 1250-1252 décrit des dérivés de l'amino-2 thiadiazole de formules

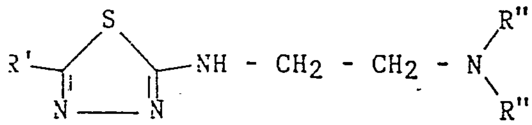

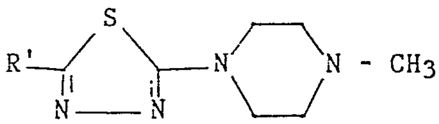

dans lesquelles :
- R′ représente un méthyle, un trifluorométhyle ou un phényle monosubstitué,et
- R″ représente un méthyle ou un éthyle.

Ces composés présentent des propriétés antithistaminiques et anticholinergiques.

Ainsi, la demande de brevet européen 183 577 et l'article précité du J. Pharm. Sci. décrivent des dérivés du thiadiazole-1, 3, 4, substitués en position 5 par un groupement phényle et en position 2 par un groupement amine.

Dans les 2 cas, le groupement amine est de la forme :

- N - X - N -

dans laquelle X est un groupement carboné linéaire ou inclus dans un système cyclique, qui comprend 2 atomes de carbone.

Dans la présente invention, nous avons trouvé de nouveaux dérivés du thiadiazole-1, 3, 4 portant en position 5 un groupement phényle, éventuellement substitué, et en position 2 un groupement amine de la forme :

- N - A - N -

dans laquelle le groupement A qui sépare les 2 atomes d'azote comprend 3 ou 4 atomes de carbone, ledit groupement étant soit linéaire, c'est-à-dire triméthylène ou tétraméthylène, soit inclus dans un cycle contenant l'un ou l'autre des atomes d'azote.

De façon tout à fait surprenante, on a trouvé que ces composés sont agonistes du système cholinergique muscarinique, alors que, à l'inverse, les composés de l'art antérieur pour lesquels le groupement X ne comprend que 2 atomes de carbone ne sont pas actifs sur le système cholinergique muscarinique.

Selon un premier aspect l'invention a pour objet en tant que produits nouveaux des dérivés du thiadiazole répondant à la formule générale :

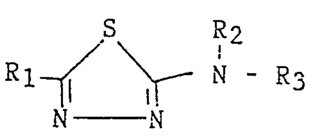

(I)

dans laquelle :

- $R_1$ représente un groupe phényle non substitué ou substitué 1 à 3 fois par un atome d'halogène, de préférence le chlore ou le fluor, par un groupe alkyle en $C_1$-$C_4$, de préférence le groupe méthyle, par un groupe alcoxy en $C_1$-$C_4$, de préférence le groupe méthoxy, par un groupe hydroxy, par un groupe trifluorométhyle ou un groupe phényle substitué à la fois par 1 à 3 atomes d'halogène et par 1 ou 2 groupements méthyle ;
- $R_2$ représente l'hydrogène ou un groupe alkyle en $C_1$-$C_4$
- $R_3$ représente :
- un groupe

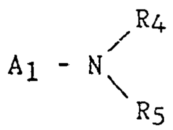

dans lequel
$A_1$ désigne un groupe alkyle droit en $C_3$ - $C_4$
$R_4$ et $R_5$ considérés indépendamment représentent l'hydrogène, un groupe alkyle en $C_1$ - $C_4$, ou encore $R_4$ et $R_5$ considérés avec l'atome d'azote auquel ils sont liés constituent un hétérocycle à 5 ou 6 chaînons contenant éventuellement un second hétéroatome et notamment l'un des cycles pyrrolidine, imidazole, pipéridine, morpholine, pipérazine ou N-alkylpipérazine ;
- un groupe

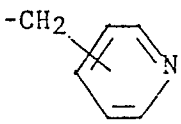

où le groupe méthylène substitue le cycle pyridine en position 3 ou 4 ;
- un groupe

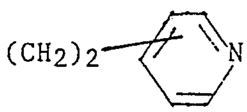

où le groupe éthylène substitue le cycle pyridinique en position 2 ou 3;
- un groupe

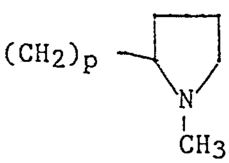

avec p = 2 ou 3 ;
- un groupe

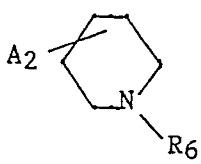

où $R_6$ désigne un groupe méthyle ou éthyle et $A_2$ désigne un groupe $(CH_2)_m$ où m = 1 ou 2, le groupe $A_2$ étant en position 3 ou 4 du cycle pipéridine lorsque m est 1, et en position 2 ou 3 de celui-ci lorsque m est 2 ;
- un groupe

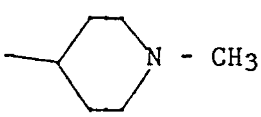

ou encore le substituant

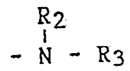

représente un groupe

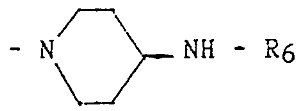

où $R_6$ est comme indiqué ci-dessus ainsi que leurs sels d'addition avec les acides minéraux ou organiques.

La présente invention se rapporte également au procédé d'obtention des composés de formule (I), procédé caractérisé en ce que l'on traite un halogéno-2 thiadiazole-1,3,4 de formule

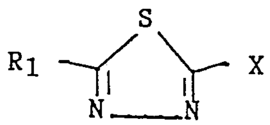

(II)

dans laquelle X représente le chlore ou le brome et $R_1$ est tel que défini ci-dessus, avec un composé de formule :

- HN $R'_2R'_3$ (III)

dans laquelle $R'_2$ et $R'_3$ ont les mêmes signififications que $R_2$ et $R_3$ définis ci-dessus ou représentent l'un ou l'autre un groupe protecteur lorsque $R_2$ et $R_3$ sont tous les deux l'hydrogène, et on transforme, éventuellement, le produit ainsi obtenu dans un de ses sels pharmaceutiquement acceptables

La réaction de substitution (II) + (III) est réalisée en présence, soit d'un réactif qui fixe l'hydracide XH formé au cours de la réaction, soit d'un excès d'amine (III), à température comprise entre 40° C et 130° C. La réaction est effectuée dans un solvant polaire tel qu'un alcanol comme l'éthanol, le n-butanol ou l'isopropanol ou dans un solvant apolaire comme le benzène. On opère de préférence sous atmosphère d'azote pour éviter la carbonatation de l'amine (I) formée.

Le produit ainsi obtenu est isolé selon les techniques classiques, par exemple par évaporation du solvant et par chromatographie du résidu, ou par évaporation du solvant et isolement de la base, éventuellement après recristallisation, ou bien par évaporation du solvant et isolement du produit sous forme d'un de ses sels par traitement du résidu, constitué par la base libre brute, avec une solution d'un acide, par exemple dans un alcool.

Lorsque le produit final est isolé sous forme de sel, la base libre peut être libérée par neutralisation.

Lorsque $R_1$ représente le groupe hydroxyphényle ou polyhydroxyphényle, les composés selon l'invention s'obtiennent par déméthylation des composés (I) dans lesquels $R_1$ représente un groupe méthoxyphényle ou polymétoxyphényle en opérant selon un procédé connu, par exemple par chauffage en milieu acide.

Les produits de départ halogénés (II) sont connus ou peuvent être préparés par des méthodes connues. Ainsi les produits (II) peuvent être obtenus à partir des composés aminés correspondants :

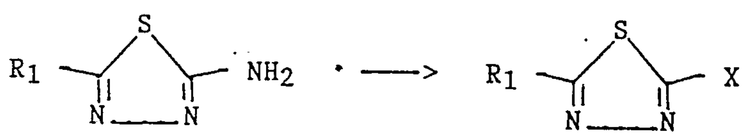

(IV) (II)

On effectue une diazotation suivie de la décomposition du sel de diazonium,en présence de l'hydracide XH, selon les méthodes décrites dans Chemische Berichte, 1956, *89*, 1534-1543 et Tetrahedon, 1968, *24*, 3209-3217.

Les amino thiadiazoles (IV) sont connus ou peuvent être préparés selon des procédés connus qui consistent à transformer l'acide $R_1COOH$ ou le chlorure d'acide $R_1COCl$ en thiosemicarbazide correspondant et à cycliser ce dernier par un agent de déshydratation selon les méthodes décrites dans J. Pharm. Soc. Japan, 1952, *72*, 373-375 ; J. Chem. Soc., 1949, 1163-1167 et Can. J. Chem., 1959, *37*, 1121-1123. Comme agent de déshydratation, on peut utiliser l'acide polyphosphorique, l'acide méthanesulfonique ou l'acide sulfurique lorsque $R_1$ représente un groupement phényle.

Les composés HN $R_2$ $R_3$ (III) sont connus ou sont préparés par des méthodes connues.

Lorsque $R_3$ représente un groupe - $A_1$ - $NR_4$ $R_5$ dans lequel $A_1$ est le tétraméthylène, on peut, soit préparer la diamine

$R_2NH$ - $(CH_2)_4$ - N $R_4$ $R_5$ (III)

par des méthodes connues, puis la faire réagir sur le dérivé du thiadiazole (II), soit obtenir le composé selon l'invention (I) par hydrogénation catalytique du dérivé acétylénique du thiadiazole (V) correspondant, selon le

schéma réactionnel suivant :

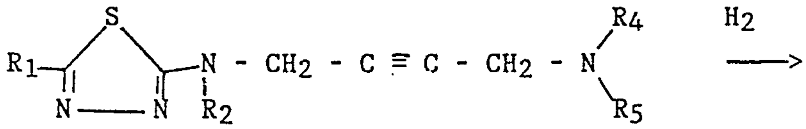

(I)

(V)

Le composé (V) peut être obtenu par substitution du thiadiazole (II) par la butyne-2 diamine-1,4 (VI) :

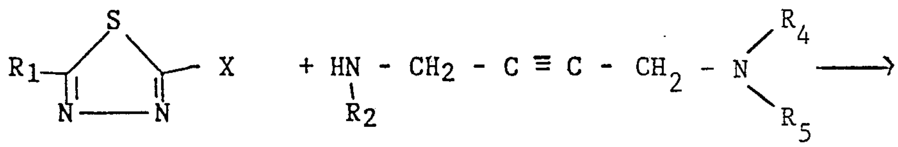
(V)

(II) (VI)

La réaction est effectuée dans des conditions semblables à celles décrites pour la substitution de (II) par le dérivé HN $R_2$ $R_3$ (III).

Les composés (VI) peuvent être préparés par différentes méthodes connues selon les valeurs des substituants $R_2$, $R_4$ et $R_5$

Par exemple, la préparation d'un composé (VI) symétrique dans lequel $R_2 = R_4 =$ alkyle et $R_5 = H$ est réalisée à partir du butyne-2 diol-1,4 par action du chlorure de thionyle en excès dans la pyridine puis action de l'amine $NH_2R_2$ sur le dérivé dichloré obtenu, selon le schéma réactionnel suivant, décrit dans J. Chem. Soc., 1946, 1007-1014 :

$HOCH_2 - C \equiv C - CH_2OH + SOCl_2$

$-> ClCH_2 - C \equiv C - CH_2Cl$

$+ NH_2R_2$

$-> R_2NH - CH_2 - C \equiv C - CH_2 - NHR_2$

Par ailleurs, la synthèse de dérivés (VI) dans lesquels $R_2$ représente l'hydrogène peut être obtenue à partir du N-propargyl phtalimide sur lequel on effectue une réaction de Mannich pour former le N(amino-4 butynyl-2) phtalimide selon le mode opératoire décrit dans Eur. J. Med. Chem. - Chim. Ther., 1982, *17*, 85-88.

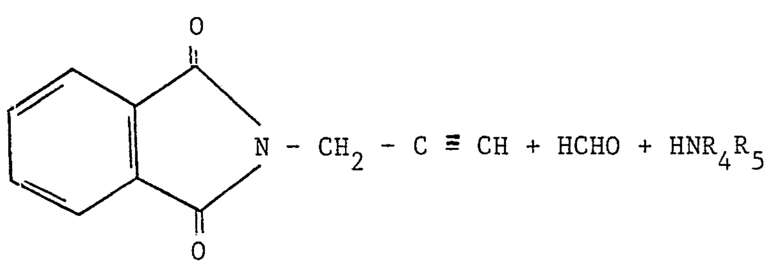

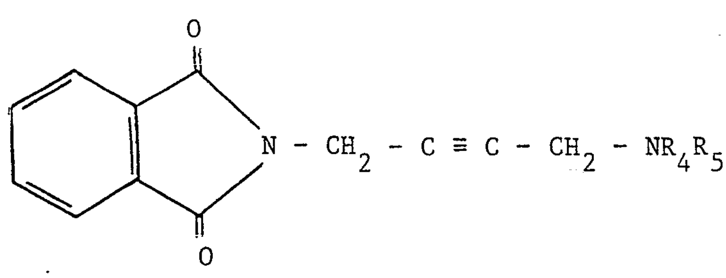

$+ NH_2 - NH_2,H_2O$

$--> \quad H_2N - CH_2 - C \equiv C - CH_2 - NR_4R_5$

Enfin, la synthèse des dérivés (VI) dans lesquels $R_2$ représente un alkyle peut également être effectuée selon la méthode décrite dans Can. J. Chem., 1980, *58*, 2183-2188 ; par aminométhylation d'une N-alkyl N-propargylphosphoramide puis hydrolyse acide du produit obtenu selon le schéma réactionnel suivant:

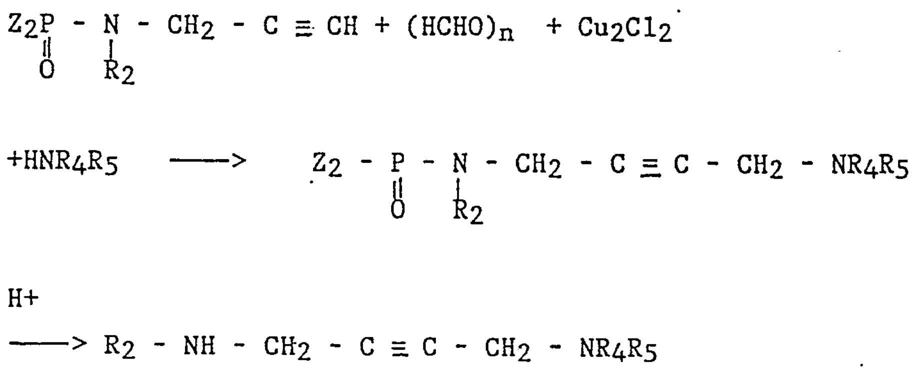

Z = alkyle

Dans le cas particulier où $R_3$ représente un groupe :

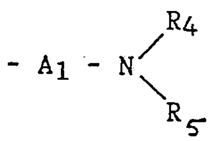

dans lequel au moins l'un des substituants $R_4$ et $R_5$ est l'hydrogène, les dérivés aminés (III) doivent être protégés Ils peuvent être préparés à partir de l'aminonitrile $R_4NH-A'_1-CN$. Celui-ci, traité par l'anhydride $(Boc)_2O$, conduit à l'aminonitrile

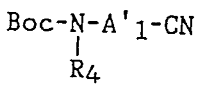

qui par réduction conduit à l'amine primaire correspondante

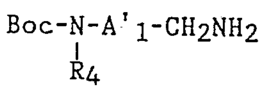 (VII)

où $A'_1$ privé d'un groupe méthylène et Boc représente le groupement tertiobutyloxycarbonyle.

Lorsque $R_2$ est différent de H, on utilise ensuite une méthode connue pour préparer la diamine convenable :

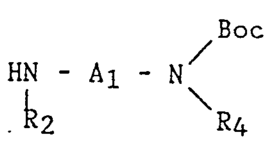 (VIII)

La réaction de substitution du thiadiazole (II) avec la diamine (VII) ou (VIII) est conduite dans les conditions habituelles. Enfin, le composé obtenu est déprotégé en milieu acide pour préparer le composé (I) selon l'invention.

Les exemples suivants illustrent l'invention sans toutefois la limiter. Dans ces exemples, le terme Boc désigne le groupement tertiobutyloxycarbonyle.

EXEMPLE 1

Dichlorohydrate de (diméthylamino-3 propylamino)-2 (triméthyl-2,4,6 phényl)-5 thiadiazole-1,3,4 : SR 45 341 A.

A) Triméthyl-2,4,6 benzoyl thiosemicarbazide.

On dissout 37,25 g de thiosemicarbazide dans 400 ml de diméthylformamide et 32,05 g de pyridine, puis on ajoute à 0°C en 20 minutes, 74 g de chlorure de triméthyl-2,4,6 benzoyle. On chauffe le mélange réactionnel pendant 2 heures à 80°C puis on concentre sous vide au trois-quart et on verse le mélange résiduel dans 1500

ml d'eau froide. On filtre le précipité blanc formé, lave à l'eau et sèche.

On obtient 78 g

Rendement : 81 %

Point de fusion supérieur à 260°C

B) Amino-2 (triméthyl-2,4,6 phényl)-5 thiadiazole-1,3,4.

On mélange 60 g de triméthyl-2,4,6 benzoyl thiosemicarbazide précédemment obtenu et 1250 g d'acide polyphosphorique. On chauffe sous agitation à 130°C pendant 18 heures puis on verse dans 20 litres d'eau froide. On filtre le précipité formé, lave à l'acétate d'éthyle et à l'éther isopropylique puis on sèche.

Poids obtenu : 22,9 g

Rendement : 41 %

Point de fusion : environ 280°C

C) Chloro-2 (triméthyl-2,4,6 phényl)-6 thiadiazole-1,3,4

On mélange dans un mortier 24,5 g d'amino-2 (triméthyl-2,4,6 phényl)-5 thiadiazole-1,3,4 et 122,5 g de nitrite de sodium. On additionne ce mélange par portions à -10°C en 2 heures 30 à une solution contenant 302 ml d'acide chlorhydrique concentré à 37 % (d = 1,19) et 5 g de cuivre. On réchauffe ensuite lentement le mélange réactionnel pour atteindre 0°C après 30 minutes et + 15°C après 1 heure. On abandonne 1 nuit à température ambiante puis on ajoute 1 litre d'eau. On extrait 2 fois par 700 ml d'acétate d'éthyle, lave par 500 ml de soude 2 N puis par 200 ml d'eau saturée en chlorure de sodium. On sèche sur sulfate de sodium, concentre puis chromatographie le résidu sur gel de silice. On élimine les impuretés de tête par le mélange chlorure de méthylène-héxane(60/40), puis le produit attendu est élué par le chlorure de méthylène.

Poids obtenu : 17,3 g

Point de fusion : 90-92°C

Rendement : 65 %

D) SR 45 341 A

On additionne 3,6 g de diméthylamino propylamine à 2,8 g de chloro-2 (triméthyl-2,4,6 phényl)-5 thiadiazole-1,3,4 dissous dans 60 ml de butanol et on chauffe à reflux pendant 18 heures sous atmosphère d'azote. On concentre puis on reprend par 200 ml d'acétate d'étyle et on extrait par 2 fois 50 ml d'acide chlorhydrique 4 N. On rend le milieu basique par addition de soude concentrée en refroidissant dans la glace puis on extrait par 2 fois 150 ml d'acétate d'éthyle, sèche sur sulfate de sodium et concentre. On effectue ensuite une chromatographie sur alumine en éluant par l'acétate d'éthyle, puis après élimination des impuretés de tête, on élue par le mélange acétate d'éthyle-méthanol (50/50) le produit attendu sous forme de base.

Poids obtenu : 2,7 g

Point de fusion : 102-104°C

Rendement : 76 %

On reprend dans 150 ml d'éthanol et on ajoute 2,26 ml d'acide chlorhydrique concentré. On laisse cristalliser puis on filtre pour obtenir le produit attendu.

Poids obtenu : 2,9 g

Rendement : 65 %

Point de fusion : 216-218°C

EXEMPLE 2

Dichlorhydrate de (diméthylamino-3 propylamino)-2 (dibromo-3,5 trimét yl-2,4,6 phényl)-5 thiadiazole-1,3,4 : SR 45 639 A.

A) Bromo-2 (bromo-5 triméthyl-2,4,6 phényl)-5 thiadiazole-1,3,4 et bromo-2 (dibromo-3,5 triméthyl-2,4,6 phényl)-5 thiadiazole-1,3,4.

On mélange dans un mortier 24,5 g d'amino-2 (triméthyl-2,4,6 phényl)-5 thiadiazole obtenu précédemment et 122,5 g de nitrite de sodium. On additionne cette composition par portions, à -30°C à une solution comprenant 402 ml d'acide bromhydrique à 47 % et 5 g de cuivre. On réchauffe le mélange lentement et progressivement de manière à ce que la température soit de -20°C après 1 heure et de + 10°C après 2 heures, puis on chauffe à 35°C pendant 2 heures. On ajoute 1 litre d'eau froide puis on extrait par 2 fois 700 ml d'acétate d'éthyle. On lave par 500 ml de soude 2 N puis par 200 ml d'eau saturée de chlorure de sodium. On sèche sur sulfate de sodium puis concentre. Le résidu est chromatographié sur gel de silice en éluant par du chlorure de méthylène.

Le premier composé élué est le bromo-2 (dibromo-3,5 triméthyl-2,4,6 phényl)-5 thiadiazole-1,3,4.

Poids obtenu : 6,2 g

Point de fusion : 131-132°C

Le second composé élué est le bromo-2 (bromo-5 triméthyl-2,4,6 phényl)-5 thiadiazole-1,3,4.

Poids obtenu : 19,3 g

Point de fusion : 91°C

B) SR 45 639 A

On chauffe à reflux sous agitation et sous atmosphère d'azote pendant 1 nuit la solution de 2,2 g du dérivé dibromé et 2,04 g de diméthylamino propylamine dans 20 ml de n-butanol. On concentre puis on reprend par 200 ml d'acétate d'éthyle. On extrait 2 fois par 100 ml d'acide chlorhydrique 4 N. On laisse décanter puis on lave par 50 ml l'acétate d'éthyle. On alcalinise par addition de 120 ml de soude concentrée en refroidissant. On extrait à l'acétate d'éthyle puis on sèche sur sulfate de sodium et concentre.

On obtient 1,9 g du produit attendu sous forme de base.

Rendement : 82 %

Point de fusion : 162-164°C

On reprend cette base dans 200 ml d'alcool absolu, on ajoute 1,06 ml d'acide chlorhydrique concentré, on filtre à chaud, concentre jusqu'à 100 ml, laisse cristalliser puis essore.

On obtient 1,8 g du produit attendu.

Point de fusion : 234-236°C

Rendement : 65 %

EXEMPLE 3

Dichlorhydrate de (diéthylamino-4 butylamino)-2 phényl-5 thiadiazole-1,3,4 : SR 45 339 A.

A) Hydrate de / (diéthylamino-4 butyne-2 yl) amino /-2 phényl-5 thiadiazole-1,3,4.

On mélange 4 g de bromo-2 phényl-5 thiadiazole-1,3,4 préparé comme indiqué dans Tetrahedron, 1968, *24*, 3214, avec 6,98 g de diéthylamino-4 butyn-2 yl amine préparé comme indiqué dans Eur. J. Med. Chem. Chim. Ther., 1982, *17*, 85-88. On chauffe à reflux dans 100 ml d'éthanol pendant 48 heures. L'éthanol est concentré sous vide, puis on reprend le résidu avec une solution de soude 4 N ; on extrait par de l'acétate d'éthyle, puis on lave la phase organique par de l'eau, on sèche sur sulfate de sodium puis on chromatographie l'huile résiduelle sur gel de silice en éluant par l'acétate d'éthyle.

Les fractions pures sont concentrées sous vide. On recristallise dans un mélange héxane-éther isopropylique (50/50). Le produit cristallise avec une molécule d'eau.

Poids : 0,26 g

Point de fusion : 55°C

B) SR 45 339 A

On effectue une hydrogénation à pression atmosphérique et température ambiante pendant 20 heures de 1,75 g du composé préparé à l'étape précédente dissous dans 100 ml d'éthanol et 1 ml d'acide chlorhydrique concentré en présence de platine préparé in situ à partir de 0,34 g d'oxyde de platine.

On purge avec de l'azote, filtre le catalyseur. On concentre le filtrat, reprend par de l'acétate d'éthyle, extrait 2 fois par 50 ml d'acide chlorhydrique 4 N. On ajoute de la soude diluée pour obtenir un pH basique (pH = 10-11). On extrait à l'acétate d'éthyle, sèche et concentre.

Poids obtenu : 1,4 g

Rendement : 80 %

On reprend la base dans 70 ml d'éthanol, ajoute 1,1 ml d'acide chlorhydrique concentré et laisse cristalliser. On essore puis lave à l'éther isopropylique.

Poids obtenu : 0,85 g

Rendement global : 39 %

Point de fusion : 158-160°C

EXEMPLE 4

Dichlorhydrate de (diméthylamino-4 butylamino)-2 (triméthyl-2,4,6 phényl)-5 thiadiazole-1,3,4 : SR 45 641 A.

La diméthylamino-4 butylamine est préparée selon la méthode reportée dans Beilstein, tome 4, supplément 3, 573-574.

Dans 60 ml de butanol normal, on ajoute 4,28 g de diméthylamino-4 butylamine et 2,2 g de chloro-2 (triméthyl-2,4,6 phényl)-5 thiadiazole préparé à l'exemple 1, étape C.

On chauffe à reflux sous atmosphère d'azote pendant 1 semaine, puis on ajoute 2,14 g de diméthylamino-4 butylamine et on continue le chauffage pendant 1 semaine. Après concentration du mélange, on reprend le résidu par l'acétate d'éthyle, extrait 2 fois par 50 ml d'acide chlorhydrique 4 N puis lave à l'acétate d'éthyle. On alcalinise par de la soude concentrée en refroidissant, puis extrait à l'acétate d'éthyle, sèche sur sulfate de sodium et concentre. Le résidu est chromatographié sur alumine ; on élimine les impuretés de tête par élution à l'acétate d'éthyle, puis on élue le produit attendu par un mélange acétate d'étyle-méthanol (80/20). Après concentration, il se forme une huile qui concrétise et le produit attendu est obtenu sous forme de base.

Poids obtenu : 1,25 g

Rendement : 39 %

Ce produit est repris dans 100 ml d'alcool absolu, on ajoute 10 ml d'acide chlorhydrique concentré, on filtre à chaud pour enlever les impuretés, puis on concentre jusqu'à un volume de 30 ml, on laisse cristalliser puis on

essore pour obtenir le sel attendu.
Poids : 0,8 g
Point de fusion : 216-218°C
Rendement global : 22 %

EXEMPLE 5

Dichlorhydrate de (méthylamino-3 propylamino)-2 (triméthyl-2,4,6, phényl)-5 thiadiazole-1,3,4 : SR 45 418 A.

A) N-phényl N-Boc amino-3 propylamine.

On verse 34 g de N-méthylamino-3 propionitrile dans 100 ml de dichlorométhane. On ajoute sous agitation 88 g de $(Boc)_2O$ et on laisse 18 heures sous agitation à température ambiante. Après concentration, on obtient 77 g de N-méthyl N-Boc amino-3 propionitrile que l'on utilise tel quel.

On mélange le produit obtenu, 800 ml d'eau, 400 ml d'éthanol et 80 ml d'ammoniaque, puis on effectue une hydrogénation catalytique sur Nickel de Raney activé pendant 10 minutes. Le volume d'hydrogène absorbé est de 16,7 litres. On concentre puis on reprend par du chlorure de méthylène, laisse décanter, sèche sur sulfate de sodium et chlorure de calcium, puis concentre. On obtient une huile qui est utilisée telle quelle à l'étape suivante.

B) SR 45 418 A

On chauffe à reflux pendant 72 heures sous azote et sous agitation dans 60 ml de butanol normal un mélange contenant2,2 g de chloro-2 (triméthyl-2,4,6, phényl)-5 thiadiazole-1,3,4, et 8,67 g de N-méthyl N-Boc amino-3 propylamine. On concentre, puis on reprend par 200 ml d'eau et extrait 2 fois par 200 ml de chlorure de méthylène. On lave à l'eau, sèche sur sulfate de sodium et concentre. On effectue une chromatographie sur gel de silice. En éluant par l'acétate d'éthyle, on élue le produit attendu. Après concentration l'huile résiduelle cristallise dans l'éther isopropylique. Le produit est sous forme d'une base dans laquelle l'azote terminal porte un groupement Boc. Il s'agit du (N-méthyl N-Boc amino-3 propylamino)-2 (triméthyl-2,4,6, phényl)-5 thiadiazole-1,3,4.
Poids obtenu : 2,2 g
Point de fusion : 113-115°C
Rendement : 65 %

On reprend cette base dans 150 ml d'alcool absolu puis on ajoute 2,9 ml d'acide chlorhydrique concentré. On concentre sous un volume de 100 ml, on laisse cristalliser puis on filtre.
On obtient 1,8 g du produit attendu.
Point de fusion : 256-258°C
Rendement global : 54 %

EXEMPLE 6

Dichlorhydrate de (méthylamino-4 pipéridino)-2 phényl-5 thiadiazole-1,3,4 : SR 44 825 A.

On dissout 2,4 g de bromo-2 phényl-5 thiadiazole-1,3,4 dans 25 ml de butanol normal, on ajoute 3,85 g de méthylamino-4 méthyl-1 pipéridine et on chauffe à reflux sous atmosphère d'azote. On refroidit, filtre le précipité et concentre le filtrat sous vide. Le résidu est repris par 100 ml d'acétate d'éthyle puis on extrait 2 fois par 50 ml d'acide chlorhydrique 3 N. On alcalinise par 50 ml de soude concentrée en refroidissant. On extrait par 2 fois 100 ml d'acétate d'éthyle, sèche sur sulfate de sodium et concentre. Le résidu est recristallisé dans un mélange chlorure de méthylène-éther isopropylique. On obtient le produit décrit sous forme de base.
Poids : 0,83 g
Point de fusion : 96°C
Rendement : 29 %

On reprend cette base dans 50 ml d'éthanol absolu. On additionne 0,74 ml d'acide chlorhydrique concentré, on filtre à chaud puis concentre au 3/4 au bain-marie et on laisse cristalliser.
Poids : 0,9 g
Rendement global : 25 %
Point de fusion > 260°C

Le spectre de RMN du SR 44 825 A a été enregistré dans le DMSO à 250 MHz.

Pour interpréter de spectre, les abréviations suivantes sont utilisées :
D = doublet, T = triplet, Q = quartet, M = multiplet, J = constante de couplage, a = axial, e = équatorial.

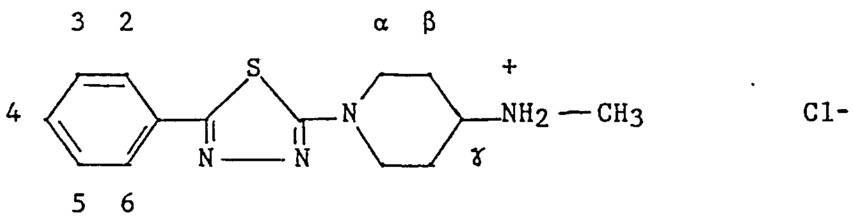

SPECTRE DE RMN

| Delta | Aspect | Protons | Attribution |
|---|---|---|---|
| 1,7 | Q de D<br>J = 4Hz<br>J = 14Hz | 2 H | Hβa |
| 2,15 | D de Q<br>J = 4Hz<br>J = 14Hz | 2 H | Hβe |
| 2,5 | T | 3 H | $CH_3$ |
| 3,25 | T de D<br>J = 14Hz<br>J = 4Hz | 2 H | Hαa |
| 4 | D de T<br>J = 14Hz<br>J = 4Hz | 2 H | Hαe |
| 7,5 | M | 3 H | H 3,4 et 5 |
| 7,75 | M | 2 H | H 2 et 6 |

EXEMPLE 7

Dichlorhydrate, hydrate de [N-méthyl N-(méthyl-1 pipéridyle-4) amino]-2 phényl-5 thiadiazole-1,3,4 : SR 44828 A.

On dissout 2,4 g de bromo-2 phényl-5 thiadiazole-1,3,4 dans 25 ml de butanol normal. On ajoute 3,85 g de méthylamino-4 méthyl-1 pipéridine et on chauffe à reflux une nuit sous atmosphère d'azote. On refroidit, filtre le précipité formé et on le reprend par 20 ml de soude 2 N. On extrait par 100 ml de chlorure de méthylène, sèche sur sulfate de sodium et concentre, puis on effectue une chromatographie sur gel de silice en éluant par un mélange chlorure de méthylène-méthanol (60/40).

On obtient 0,77 g du produit attendu sous forme de base.

Point de fusion : 74-76°C

Rendement : 27 %

On dissout cette base dans 50 ml d'éthanol absolu, additionne 0,62 ml d'acide chlorhydrique concentré, filtre à chaud l'insoluble, concentre au 3/4 au bain-marie et on laisse cristalliser.

Poids obtenu : 0,65 g

Rendement : 19 %

Point de fusion : 276-278°C

EXEMPLE 8

Dichlorhydrate, hydrate de (pyridyl-4) méthylamino -2 (triméthyl-2,4,6 phényl)-5 thiadiazole-1,3,4 : SR 45 638 A.

Dans 20 ml de butanol normal, on mélange 2,2 g de chloro-2 (triméthyl-2,4,6 phényl)-5 thiadiazole-1,3,4 et 4,98 g d'aminométhyl-4 pyridine. On chauffe à reflux pendant 48 heures sous atmosphère d'azote. On concentre le milieu réactionnel puis on reprend par 30 ml d'eau et on extrait 2 fois par 50 ml de chlorure de méthylène, on sèche sur sulfate de sodium et concentre. Le résidu est chromatographié sur alumine en éluant par l'acétate d'éthyle. Après élimination des impuretés de tête, on concentre les fractions suivantes puis cristallise l'huile résiduelle.

On obtient 1,6 g du produit attendu sous forme de base.

Point de fusion : 136-138°C

Rendement : 56 %

On reprend la base dans 100 ml d'éthanol absolu, ajoute 1,3 ml d'acide chlorhydrique concentré. On filtre

l'insoluble puis concentre le filtrat jusqu'à un volume total de 30 ml. On laisse cristalliser puis essore pour obtenir le produit attendu.
Poids : 1,5 g
Point de fusion : 200-202° C
Rendement global : 41%

EXEMPLE 9

Dichlorhydrate de (diméthylamino-3 propylamino)-2 (diméthoxy-3,4 phényl)-5 thiadiazole-1,3,4 : SR 45 642 A.

On chauffe à reflux pendant une nuit sous atmosphère d'azote un mélange comprenant 4 g de chloro-2 (diméthoxy-3,4 phényl)-5 thiadiazole-1,3,4 et 6,36 g de diméthylamino-3 propylamine dans 30 ml de butanol normal. On concentre puis on reprend par 100 ml d'acétate d'éthyle et on extrait 2 fois par 70 ml d'acide chlorhydrique 4 N. On ajoute 90 ml de soude concentrée en refroidissant, on filtre le précipité formé et on lave à l'eau. Le précipité est dissous dans du chlorure de méthylène, puis on sèche sur sulfate de sodium et concentre.

Le produit attendu est obtenu sous forme de base.
Poids : 4 g
Rendement : 80 %
Point du fusion : 147-149° C

On reprend 2,25 g de cette base dans 100 ml d'éthanol absolu et on ajoute 1,78 ml d'acide chlorhydrique concentré. On filtre à chaud les impuretés. On concentre le filtrat jusqu'à un volume de 30 ml, puis on laisse cristalliser et filtre pour obtenir le sel attendu.
Poids : 2,4 g
Point de fusion : 210-212° C
Rendement : 66 %

EXEMPLE 10

Dibromhydrate, hydrate de (diméthyl amino-3 propylamino)-2 (dihydroxy-3,4 phényl)-5 thiadiazole-1,3,4 : SR 45 643 A.

On chauffe à reflux pendant 7 heures un mélange contenant 1,75 g de SR 45 642 à l'état de base et 25 ml d'acide bromhydrique à 47 %. On concentre sous vide puis on reprend par 150 ml d'éthanol absolu à chaud. On filtre puis concentre jusqu'à un volume de 50 ml. On laisse cristalliser puis filtre pour obtenir le produit attendu.
Poids : 2,45 g
Rendement : 95 %
Point de fusion : 172-174° C

EXEMPLE 11

Dichlorhydrate de [ (N -méthyl pipéridyle-3) méthyl amino) ]-2 (chloro-4 phényl)-5 thiadiazole-1,3,4 : SR 45 491 A.

A) N-méthyl aminométhyl-3 pipéridine.

Ce composé est préparé à partir de l'aminométhyl-3 pyridine selon la méthode décrite dans Biochem. J., 1971, *122*, 557-567.

B) Chloro-2 (chloro-4 phényl)-5 thiadiazole-1,3,4.

La préparation de ce composé est décrite dans le brevet US 4 454 147.
Point de fusion : 122° C

C) SR 45 491 A.

Ce composé est obtenu à partir des produits obtenus à l'étape A et l'étape B en suivant la méthode décrite dans les exemples précédents.

Le chlorhydrate est formé dans le mélange de solvant éther éthylique-propanol-2.
Point de fusion : 178° C

En utilisant des modes opératoires semblables à ceux décrits ci-dessus, d'autres composés (I) selon l'invention ont été préparés. Ils sont décrits dans le tableau 1 ci-dessous et caractérisés par leur point de fusion (Fc).

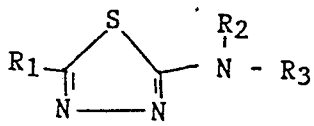

(I)

TABLEAU 1

| N° Produit | N° Exemp | $R_1$ | $R_3$ | Fc °C (solvant) |
|---|---|---|---|---|
| SR 44824 A 2HCl | 12 | phényl | diméthylamino-3 propyl | 230-232 EtOH |
| SR 45340 A 2HCl, $H_2O$ | 13 | bromo-5 triméthyl-2,4,6 phényl | diméthylamino-3 propyl | 218-220 EtOH |
| SR 45352 A 2HCl | 14 | triméthyl-2,4,6 phényl | diéthylamimo-3 propyl | 188-190 EtOH |
| SR 45417 A 2HCl | 15 | triméthyl-2,4,6 phényl | pyridyl-3 méthyl | 190-192 EtOH |
| SR 45421 A 2HCl | 16 | fluoro-4 phényl | diméthylamino-3 propyl | 233-235 iPrOH-$Et_2O$ |
| SR 45422 A 2HCl | 17 | dichloro-2,4 phényl | diméthylamino-3 propyl | 230-231 $Et_2O$ |
| SR 45447 A 2HCl 0,5 $H_2O$ | 18 | fluoro-4 phényl | pyridyl-3 méthyl | 183-184 iPrOH-$Et_2O$ |
| SR 45458 A 2HCl | 19 | dichloro-2,4 phényl | pyrrolidino-3 propyl | 230 iPrOH |

| | | | | |
|---|---|---|---|---|
| SR 45459 A<br>2HCl | 20 | dichloro-2,4<br>phényl | pyridyl-3<br>méthyl | 228<br>iPrOH |
| SR 45460 A<br>HCl | 21 | dichloro-2,4<br>phényl | pyridyl-4<br>méthyl | 246<br>iPrOH |
| SR 45479 A<br>2HCl | 22 | dichloro-2,4<br>phényl | méthylamino-3<br>propyl | 237<br>iPrHO |
| SR 45480 A<br>2HCl | 23 | chloro-4<br>phényl | diméthylamino-3<br>propyl | 238<br>iPrOH |
| SR 45481 A<br>2HCl | 24 | dichloro-2,4<br>phényl | diéthylamino-3<br>propyl | 122<br>iPrOH |
| SR 45482 A<br>HCl | 25 | fluoro-4<br>phényl | pyridyl-4<br>méthyl | 222-224<br>iPrOH-$Et_2O$ |
| SR 45483 A<br>2HCl<br>1,5 $H_2O$ * | 26 | fluoro-4<br>phényl | diméthylamino-3<br>propyl | 180-182<br>iPrOH-$Et_2O$ |
| SR 45488 A | 27 | chloro-4<br>phényl | pyridyl-3<br>méthyl | 208<br>$Et_2O$ |
| SR 45489 A<br>3 HCl | 28 | chloro-4<br>phényl | (méthyl-4<br>pipérazinyl-1)-3<br>propyl | 256-257<br>iPrOH |
| SR 45490 A<br>2HCl<br>0,5 $H_2O$ | 29 | dichloro-2,4<br>phényl | (méthyl-1 pyrro-<br>lidinyl-2)-2<br>éthyl | 228<br>iPrOH |

| | | | | |
|---|---|---|---|---|
| SR 45492 A 2HCl | 30 | dichloro-2,4 phényl | (pyridyl-2)-2 éthyl | 212 iPrOH |
| SR 45504 2HCl | 31 | dichloro-2,4 phényl | (imidazolyl-1)-3 propyl | 258 iPrOH |
| SR 45520 A 2HCl,$H_2O$ | 32 | chloro-4 phényl | morpholino-3 propyl | 242 iPrOH |
| SR 45530 A 2HCl * | 33 | fluoro-4 phényl | pyridyl-3 méthyl | 254 iPrOH |
| SR 45544 A HCl 0,5 $H_2O$ | 34 | chloro-4 phényl | pyridyl-4 méthyl | 244 iPrOH |
| SR 45640 A 2HCl | 35 | trifluoro-méthyl-4 phényl | diméthylamino-3 propyl | 220-222 EtOH |
| SR 45716 A 2HCl | 36 | trifluoro-méthyl-3 phényl | diméthylamino-3 propyl | 198-200 EtOH |
| SR 45717 2HCl | 37 | chloro-2 phényl | diméthylamino-3 propyl | 244-246 EtOH |
| SR 45718 2HCl | 38 | choro-3 phényl | diméthylamino-3 propyl | 215-217 |

* Le radical $R_2$ représente l'hydrogène, sauf pour les composés :

SR 45483 A où $R_2$ est un éthyle

SR 45530 A où $R_2$ est un méthyle

Les abréviations suivantes ont été utilisées pour les solvants de recristallisation : EtOH : éthanol, iPrOH : isopropanol, $Et_2O$ : éther éthylique.

Le point de fusion (Fc) de certains composés sous forme de base a été mesuré après leur purification par chromatographie :

Exemple n°

| | |
|---|---|
| 12 | Fc = 66- 68°C |
| 14 | Fc = 62- 64°C |
| 15 | Fc = 130-132°C |
| 35 | Fc = 148-150°C |
| 36 | Fc = 79- 81°C |
| 37 | Fc = 62- 64°C |
| 38 | Fc = 76- 78°C |

Les produits selon l'invention ont été étudiés en ce qui concerne leurs propriétés pharmacologiques et en particulier en ce qui concerne leur affinité pour les récepteurs cholinergiques muscariniques.

L'étude a été effectuée par des tests biochimiques in vitro et également par des tests pharmacologiques réalisés chez l'animal.

*ETUDE BIOCHIMIQUE IN VITRO*

Chez les mammifères, il existe deux sous classes de récepteurs cholinergiques muscariniques : les récepteurs $M_1$ et $M_2$

Les récepteurs de type $M_1$ sont concentrés dans certaines zones de cerveau telles que l'hippocampe, le cortex cérébral, le striatum ainsi que dans les ganglions sympathiques. Ces sites de liaison peuvent être sélectivement marqués par la pirenzépine tritiée ($^3$H-PZ). Ceux de type $M_2$ prédominent dans le coeur et dans l'iléon et peuvent être marqués par le N-méthylscopolamine tritiée ($^3$H-NMS). Afin de déterminer la sélectivité des produits de l'invention, vis-à-vis des sites $M_1$ et $M_2$, on a étudié leur intéraction in vitro avec les fixations à haute affinité de la $^3$H-PZ et de la$^3$H-NMS sur des membranes d'hippocampe de rat et de muscle lisse d'iléon de cobaye, respectivement.

Méthodologies

a) Recherche d'une affinité pour le récepteur cholinergique muscarinique de type $M_1$

L'intéraction des molécules avec les récepteurs muscariniques de type $M_1$ a été étudiée en mesurant in vitro sur un homogénat d'hippocampe de rat, le déplacement de la pirenzépine tritiée de ses sites de fixation spécifiques. Des aliquots (10 µl) d'un homogénat d'hippocampe de rat à 5 % (P/v) dans un tampon $Na_2HPO_4$ (50 mM, pH 7,40), sont incubés 2 heures à 4°C en présence de$^3$H-PZ (76 Ci/mmole 1 nM final) et de concentrations croissantes en produit à étudier. Le volume final est de 2 ml. La réaction est arrêtée par centrifugation 10 minutes à 50.000 xg. Après décantation et lavage des culots, la radioactivité fixée est comptée par scintillation liquide. La fixation non spécifique est déterminée en présence de 10 µM de sulfate d'atropine. La concentration inhibitrice 50 ($CI_{50}$) est déterminée graphiquement. (Réf. : Watson J.D., Roeskoe W.R. and Yamamura H.I., Life Sci., 1982, *31*, 2019-2029).

b) Recherche d'une affinité pour le récepteur cholinergique muscarinique de type $M_2$.

L'intéraction avec les récepteurs muscariniques de type $M_2$ a été étudiée en mesurant in vitro sur homogénat de muscle lisse d'iléon de cobaye, le déplacement de la N-méthyl-scopolamine tritiée de ses sites de fixation spécifiques. Des aliquots (50 µl) d'un homogénat de muscle lisse d'iléon de cobaye à 0,625 % P/v) dans 20 mM de tampon HEPES : acide ((hydroxy-2 éthyl-4) pipérazine-1-yl)-2 éthanesulfonique, contenant NaCl (100 mM) et Mg $Cl_2$ (10 mM) (pH final : 7,5), sont incubés 20 minutes à 30°C en présence de $^3$H-NMS (85 Ci/mmole 0,3 nM final) et de concentrations croissantes en produits à tester. Le volume final est de 1 ml. La réaction est arrêtée par centrifugation 5 minutes à 15.000 xg. La fixation non spécifique est déterminée en présence de 10 mM de sulfate d'atropine. (Réf. : Hammer R. et al., Nature, 1980, *283*, 90-92 ; Hulme E.C. et al., Mol. Pharmacol., 1978, *14*, 737-750).

Résultats

Le tableau 2 indique les affinités des produits de l'invention pour les récepteurs $M_1$ et $M_2$. Les résultats sont exprimés en concentrations inhibitrices 50 pour cent ($CI_{50}$), soit la concentration (en µM) qui induit un déplacement de 50 % du ligand tritié fixé aux récepteurs membranaires. La $CI_{50}$ de déplacement de la$^3$H-PZ représente l'affinité pour le récepteur $M_1$

la $CI_{50}$ de déplacement de la$^3$H-NMS représente l'affinité pour le récepteur $M_2$.

De plus, dans la colonne suivante du tableau, on a indiqué le rapport R entre les $CI_{50}$ pour les récepteurs $M_1$ et $M_2$ qui exprime la sélectivité des produits vis-à-vis de l'un des types de récepteurs.

TABLEAU 2

| N° de Produit | $^3$H-PZ($M_1$) $CI_{50}$ µM | $^3$H-NMS($M_2$) $CI_{50}$ µM | R = $CI_{50}(M_2)$ / $CI_{50}(M_1)$ |
|---|---|---|---|
| SR 44824 A | 3 | > 100 | > 30 |
| SR 44825 A | 2,5 | 80 | 32 |
| SR 44828 A | 9 | - | - |
| SR 45339 A | 5 | 34 | 7 |
| SR 45340 A | 3,3 | 27 | 8 |
| SR 45341 A | 0,45 | 72 | 160 |
| SR 45352 A | 5,5 | 30 | 5 |
| SR 45418 A | 20 | > 100 | > 5 |
| SR 45422 A | 0,70 | 26 | 37 |
| SR 45458 A | 0,8 | 12 | 15 |
| SR 45479 A | 14 | 70 | 5 |
| SR 45480 A | 4,5 | 50 | 11 |
| SR 45483 A | 2,4 | 20 | 8 |
| SR 45489 A | 3,6 | 26 | 7 |
| SR 45504 A | 3 | 40 | 13 |
| SR 45520 A | 4,5 | 80 | 17 |

Les résultats montrent que les composés selon l'invention présentent une forte affinité pour les récepteurs cholinergiques muscariniques avec une spécificité marquée pour les récepteurs centraux de type $M_1$.

*ETUDE PHARMACOLOGIQUE IN VIVO*

La pirenzépine (PZ) est un antagoniste spécifique des récepteurs cholinergiques muscariniques centraux $M_1$. L'injection intrastriatale de PZ chez la souris induit un comportement rotatoire. On a étudié l'antagonisme de ce comportement par les produits selon l'invention.

Les produits selon l'invention sont administrés à la dose de 0,3 ou 3 mg/kg par voie orale (p.o.) après avoir été solubilisés dans l'eau distillée ou mis en suspension dans une solution de gomme arabique à 5 %. Les contrôles sont réalisés par administration du solvant pur dans les mêmes conditions.

Les animaux utilisés sont des souris femelles (Swiss, CD 1, Charles River, France) de poids corporel compris entre 25 et 30 grammes.

La pirenzépine est mise en solution dans un tampon phosphate, le pH de la solution est 6.

Les produits à étudier ou leur solvants sont administrés par voie orale, par une sonde oesophagienne, sous un volume de 0,4 ml pour 20 g de poids corporel. L'administration a lieu 4 heures avant une injection directe de pirenzépine à la dose de 1µg dans 1µl de solvant dans le striatum droit de la souris, selon la méthode décrite par P. WORMS et al. dans Psychopharmacology, 1987, *93*, 489-493.

Le nombre de rotations contralatérales (sens opposé au coté de l'injection) a été compté pendant trois périodes de 2 minutes après injection de pirenzépine (minutes 2 à 4, 8 à 10 et 13 à 15) puis cumulé pour chaque animal. Chaque traitement comporte 3 à 4 doses et 10 animaux par dose. Pour chaque traitement, on calcule le nombre total de rotations et le pourcentage d'inhibition vis-à-vis du lot contrôle (solvant).

Les résultats sont reportés dans le tableau 3.

A titre de comparaison, on a reporté les résultats obtenus avec certains produits connus de l'art antérieur.

Produit A : 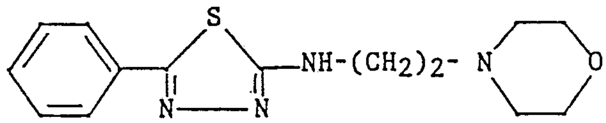 , 2 HCl

Produit B : 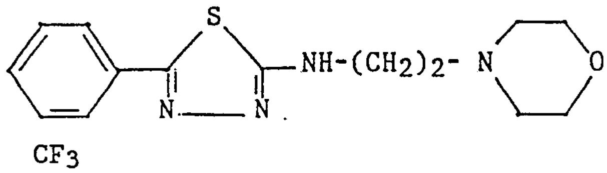 , 2 HCl

Produit C : 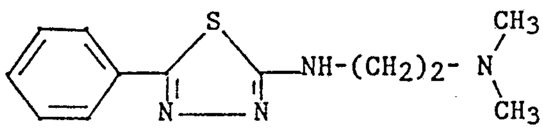 , 2 HCl

TABLEAU 3

| Produits n° | % d'inhibition | |
|---|---|---|
| | 0,3 mg/kg p.o. | 3 mg/kg p.o. |
| SR 44824 A | - 7 | - 27 ** |
| SR 44828 A | - 3 | - 26 ** |
| SR 45339 A | - 49 ** | - 77 ** |
| SR 45340 A | - 58 ** | - 95 ** |
| SR 45341 A | - 31 ** | - 74 ** |
| SR 45352 A | - 6 | - 32 ** |
| SR 45417 A | - 7 | - 43 ** |
| SR 45418 A | - 31 ** | - 77 ** |
| SR 45421 A | - 10 | - 38 ** |
| SR 45422 A | - 7 | - 50 ** |
| SR 45447 A | - 36 ** | - 62 ** |
| SR 45458 A | 0 | - 28 ** |
| SR 45459 A | - 11 | - 29 ** |
| SR 45480 A | - 35 ** | - 54 ** |
| SR 45481 A | - 31 ** | - 67 ** |
| SR 45482 A | - 23 * | - 53 ** |
| SR 45483 A | 0 | - 20 * |
| SR 45488 A | - 19 | - 49 ** |
| SR 45489 A | 0 | - 29 ** |
| SR 45490 A | - 47 ** | - 73 ** |
| SR 45491 A | - 6 | - 57 ** |
| SR 45492 A | - 12 | - 50 ** |
| SR 45504 A | - 27 * | - 60 ** |
| SR 45520 A | - 34 ** | - 75 ** |
| SR 45530 A | - 6 | - 41 ** |
| A | - 8 | - 8 |
| B | inactif | inactif |
| C | - 6 | - 12 |

* $p < 0,05$ dans le test de Student.
** $p < 0,01$ dans le test de Student.

Les résultats du tableau 3 montrent que les composés selon l'invention possèdent une activité stimulante de la transmission cholinergique centrale et sont donc susceptibles d'être utilisés comme agonistes des récepteurs muscariniques.

On constate que les produits de comparaison ne sont pas actifs sur la transmission cholinergique centrale.

Les produits selon l'invention ne manifestent aucun signe de toxicité aux doses où ils sont actifs.

La toxicité aiguë a été déterminée pour un produit selon l'invention : le SR 45341 A. Le produit a été administré par voie orale à doses croissantes à un lot de 10 souris femelles (Swiss, CD 1, Charles River, France) de poids corporel 20 g.

La mortalité provoquée par le produit étudié a été notée pendant les 24 heures suivant l'administration du produit. On a déterminé la dose léthale 50 ($DL_0$), c'est à dire la dose provoquant la mort de 50 % des animaux elle est supérieure à 300 mg/kg.

Par suite les composés (I) peuvent être utilisés en tant que médicaments.

Les résultats indiqués ci-dessus permettent d'envisager l'utilisation des produits selon l'invention pour le traitement des syndromes dégénératifs liés à la sénescence et notamment les troubles de la mémoire et les démences séniles.

Selon un autre de ses aspects la présente invention concerne donc les compositions pharmaceutiques contenant au moins un des composés de formule (I) ou un de leurs sels en tant qu'ingrédient actif.

Dans les compositions pharmaceutiques de la présente invention pour l'administration orale, sublinguale, transdermique ou rectale, les ingrédients actifs de formule (I) ci-dessus peuvent être administrés sous forme unitaires d'administration, en mélange avec les supports pharmaceutiques classiques. Les formes unitaires d'administration appropriées comprennent les formes par voie orale, telles que les comprimés, les gélules, les poudres, les granulés et les solutions ou suspensions orales, les formes d'administration sublinguale et buccale, les formes d'administration sous-cutanée, intramusculaire ou intraveineuse et les formes d'administration rectale.

Afin d'obtenir l'effet désiré, la dose de principe actif peut varier entre 20 et 500 mg par jour.

Chaque dose unitaire peut contenir de 5 à 200 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granulés dispersibles dans l'eau peuvent contenir l'ingrédient actif en mélange avec des agents de dispersion ou des agents mouillants, ou des agents de mise en suspension comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

A titre d'exemple de préparation galénique on peut préparer des gélules contenant :

SR 45341 A 0,010 g

Lactose 0,050 g

Stéarate de magnésium 0,005 g

en mélangeant intimement les ingrédients ci-dessus et en versant le mélange dans des gélules de gélatine dure.

**Revendications**

1. Dérivés du thiadiazole de formule :

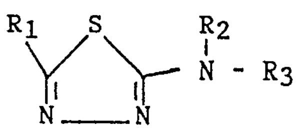

(I)

dans laquelle :

- $R_1$ représente un groupe phényle non substitué ou substitué 1 à 3 fois par un atome d'halogène, de préférence le chlore ou le fluor, par un groupe alkyle en $C_1$-$C_4$, de préférence le groupe méthyle, par un groupe alcoxy en $C_1$-$C_4$, de préférence le groupe méthoxy, par un groupe hydroxy, par un groupe trifluorométhyle ou un groupe phényle substitué à la fois par 1 à 3 atomes d'halogène et par 1 ou 2 groupements méthyle ;
- $R_2$ représente l'hydrogène ou un groupe alkyle en $C_1$-$C_4$
- $R_3$ représente :
- un groupe

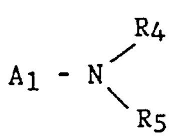

dans lequel

$A_1$ désigne un groupe alkyle droit en $C_3$-$C_4$

$R_4$ et $R_5$ considérés indépendamment représentent l'hydrogène, un groupe alkyle en $C_1$-$C_4$, ou encore $R_4$ et $R_5$ considérés avec l'atome d'azote auquel ils sont liés constituent un hétérocycle à 5 ou 6 chaînons contenant éventuellement un second hétéroatome et notamment l'un des cycles pyrrolidine, imidazole, pipéridine, morpholine, pipérazine ou N-alkylpipérazine ;

- un groupe

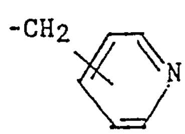

où le groupe méthylène substitue le cycle pyridine en position 3 ou 4 ;

- un groupe

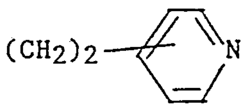

où le groupe éthylène substitue le cycle pyridinique en position 2 ou 3 ;
- un groupe

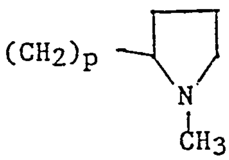

avec p = 2 ou 3 ;
- un groupe

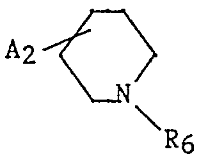

où $R_6$ désigne un groupe méthyle ou éthyle et $A_2$ désigne un groupe $(CH_2)_m$ où m = 1 ou 2, le groupe $A_2$ étant en position 3 ou 4 du cycle pipéridine lorsque m est 1, et en position 2 ou 3 de celui-ci lorsque m est 2.
- un groupe

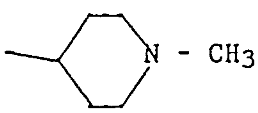

ou encore le substituant

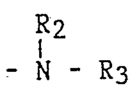

représente un groupe

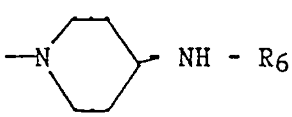

où $R_6$ est comme indiqué ci-dessus ainsi que leurs sels d'addition avec les acides minéraux ou organiques.

2. Composés selon la revendication 1 où $R_3$ représente :
- un groupe

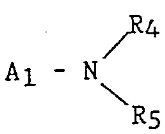

dans lequel
$A_1$ désigne un groupe alkyle droit en $C_3$ - $C_4$
$R_4$ et $R_5$ considérés indépendamment représentent l'hydrogène, un groupe alkyle en $C_1$-$C_4$, ou encore $R_4$ et $R_5$ considérés avec l'atome d'azote auquel ils sont liés constituent un hétérocycle à 5 ou 6 chaînons contenant éventuellement un second hétéroatome et notamment l'un des cycles pyrrolidine, imidazole, pipéridine, morpholine, pipérazine ou N-alkylpipérazine.

3. Composés selon la revendication 1 où $R_3$ représente :

- un groupe

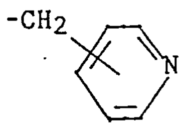

où le groupe méthylène substitue le cycle pyridine en position 3 ou 4 ;

4. Composés selon la revendication 1 où $R_3$ représente :
- un groupe

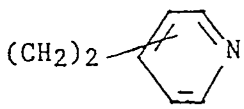

où le groupe éthylène substitue le cycle pyridinique en position 2 ou 3 ;

5. Composés selon la revendication 1 où $R_3$ représente :
- un groupe

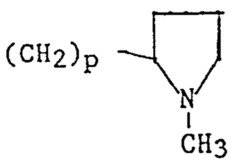

avec p = 2 ou 3.

6. Composés selon la revendication 1 où $R_3$ représente
- un groupe

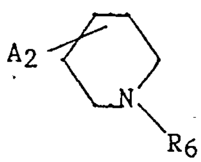

où $A_2$ désigne un groupe $(CH_2)m$ avec m = 1 ou 2, et
$R_6$ désigne un groupe méthyle ou éthyle.

7. Composés selon la revendication 1 où $R_3$ représente :
- un groupe

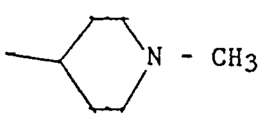

8. Composés selon la revendication 1 où le substituant

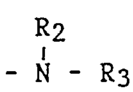

représente un groupe :

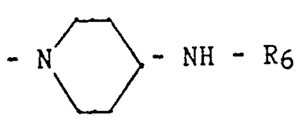

où $R_6$ désigne un groupe méthyle ou éthyle

9. Procédé d'obtention des composés de formule (I) selon la revendication 1, caractérisé en ce que l'on traite un halogéno-2 thiadiazole-1,3,4 correspondant de formule :

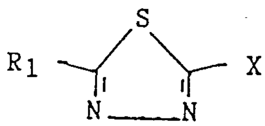

(II)

dans laquelle X représente le chlore ou le brome et $R_1$ est tel que défini dans la revendication 1, avec un composé de formule :

- $HN\,R'_2R'_3$ (III)

dans laquelle $R'_2$ et $R'_3$ ont les mêmes significations que $R_2$ et $R_3$ définis ci-dessus ou représentent l'un ou l'autre un groupe protecteur lorsque $R_2$ et $R_3$ sont tous les deux l'hydrogène et on transforme, éventuellement, le produit ainsi obtenu dans un de ses sels pharmaceutiquement acceptables.

10. Procédé selon la revendication 9, caractérisé en ce que la réaction de substitution (II) + (III) est réalisée en présence, soit d'un réactif qui fixe l'hydracide XH formé au cours de la réaction, soit d'un exès d'amine (III), à température comprise entre 40° C et 130° C, dans un solvant polaire ou apolaire.

11. Procédé d'obtention des composés de formule (I) selon la revendication 1, dans laquelle $R_1$ représente le groupe hydroxyphényle ou polyhydroxyphényle, caractérisé en ce qu'il consiste à soumettre à une déméthylation les composés de formule (I) dans lesquels $R_1$ représente un groupe méthoxyphényle ou polyméthoxyphényle obtenus par le procédé selon l'une des revendications 9 ou 10.

12. Procédé d'obtention des composés de formule (I), selon la revendication 1, dans laquelle $R_3$ représente le groupe $A_1$-$NR_4R_5$ dans lequel $A_1$ est le groupe tétraméthylène, caractérisé en ce qu'il consiste à soumettre à une hydrogénation catalytique le dérivé acétylénique du thiadiazole de formule V:

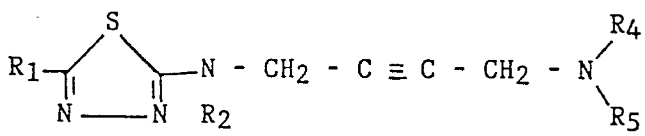

(V)

dans laquelle $R_1$, $R_2$, $R_4$ et $R_5$ sont tels que définis dans la revendication 1.

13. Compostions pharmaceutiques contenant à titre de principe actif un composé de formule (I) selon la revendication 1 ou l'un de ses sels pharmaceutiquement acceptables en combinaison avec un véhicule pharmaceutiquement acceptable.

14. Compositions pharmaceutiques selon la reventication 13 contenant de 20 à 500 mg de principe actif.

**Revendications pour l'Etat contractant suivant: ES**

1. Procédé pour l'obtention de dérivés du thiadiazole de formule :

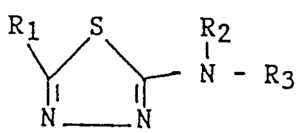

(I)

dans laquelle :

- $R_1$ représente un groupe phényle non substitué ou substitué 1 à 3 fois par un atome d'halogène, de préférence le chlore ou le fluor, par un groupe alkyle en $C_1$-$C_4$, de préférence le groupe méthyle, par un groupe alcoxy en $C_1$-$C_4$, de préférence le groupe méthoxy, par un groupe hydroxy, par un groupe trifluorométhyle ou un groupe phényle substitué à la fois par 1 à 3 atomes d'halogène et par 1 ou 2 groupements méthyle ;
- $R_2$ représente l'hydrogène ou un groupe alkyle en $C_1$-$C_4$
- $R_3$ représente :
- un groupe

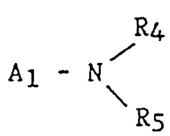

dans lequel

$A_1$ désigne un groupe alkyle droit en $C_3$-$C_4$

$R_4$ et $R_5$ considérés indépendamment représentent l'hydrogène, un groupe alkyle en $C_1$-$C_4$, ou encore $R_4$ et $R_5$ considérés avec l'atome d'azote auquel ils sont liés constituent un hétérocycle à 5 ou 6 chaînons

contenant éventuellement un second hétéroatome et notamment l'un des cycles pyrrolidine, imidazole, pipéridine, morpholine, pipérazine ou N-alkylpipérazine ;

- un groupe

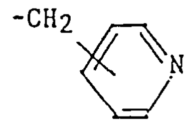

où le groupe méthylène substitue le cycle pyridine en position 3 ou 4 ;

- un groupe

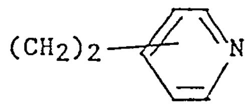

où le groupe éthylène substitue le cycle pyridinique en position 2 ou 3 ;

- un groupe

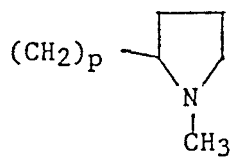

avec p = 2 ou 3 ;

- un groupe

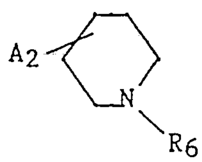

où $R_6$ désigne un groupe méthyle ou éthyle et $A_2$ désigne un groupe $(CH_2)_m$ où m = 1 ou 2, le groupe $A_2$ étant en position 3 ou 4 du cycle pipéridine lorsque m est 1, et en position 2 ou 3 de celui-ci lorsque m est 2.

- un groupe

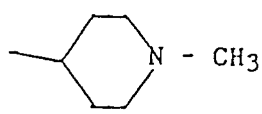

ou encore le substituant

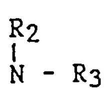

représente un groupe

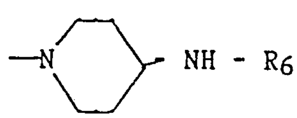

où $R_6$ est comme indiqué ci-dessus ainsi que leurs sels d'addition avec les acides minéraux ou organiques caractérisé en ce que l'on traite un halogéno-2 thiadiazole-1,3,4 correspondant de formule :

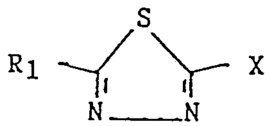

(II)

dans laquelle X représente le chlore ou le brome et $R_1$ est tel que défini dans la revendication 1, avec un composé de formule :

- HN $R'_2R'_3$ (III)

dans laquelle $R'_2$ et $R'_3$ ont les mêmes significations que $R_2$ et $R_3$ définis ci-dessus ou représentent l'un ou l'autre un groupe protecteur lorsque $R_2$ et $R_3$ sont tous les deux l'hydrogène et on transforme, éventuellement, le produit ainsi obtenu dans un de ses sels pharmaceutiquement acceptables.

2. Procédé selon la revendication 1, caractérisé en ce que la réaction de substitution (II) + (III) est réalisée en présence, soit d'un réactif qui fixe l'hydracide XH formé au cours de la réaction, soit d'un exès d'amine (III), à température comprise entre 40° C et 130° C, dans un solvant polaire ou apolaire.

3. Procédé pour l'obtention des composés de formule (I) selon la revendication 1, dans laquelle $R_1$ représente le groupe hydroxyphényle ou polyhydroxyphényle, caractérisé en ce qu'il consiste à soumettre à une déméthylation les composés de formule (I) dans lesquels $R_1$ représente un groupe méthoxyphényle ou polyméthoxyphényle obtenus par le procédé selon l'une des revendications 1 ou 2.

4. Procédé pour l'obtention des composés de formule (I), selon la revendication 1, dans laquelle $R_3$ représente le groupe $A_1$-$NR_4R_5$ dans lequel $A_1$ est le groupe tétraméthylène, caractérisé en ce qu'il consiste à soumettre à une hydrogénation catalytique le dérivé acétylénique du thiadiazole de formule V:

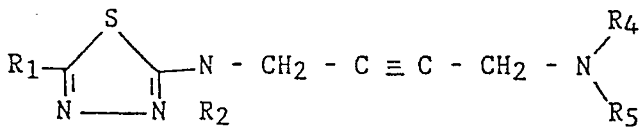

(V)

dans laquelle $R_1$, $R_2$, $R_4$ et $R_5$ sont tels que définis dans la revendication 1.

5. Utilisation des dérivés préparés par le procédé selon l'une quelconque des revendications 1 à 4, pour la préparation de médicaments agonistes du système cholinergique muscarinique.

**Revendications par l'Etat contractant suivant: GR**

1. Dérivés du thiadiazole de formule :

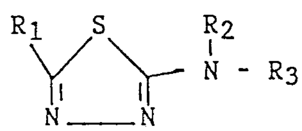

(I)

dans laquelle :

- $R_1$ représente un groupe phényle non substitué ou substitué 1 à 3 fois par un atome d'halogène, de préférence le chlore ou le fluor, par un groupe alkyle en $C_1$-$C_4$, de préférence le groupe méthyle, par un groupe alcoxy en $C_1$-$C_4$, de préférence le groupe méthoxy, par un groupe hydroxy, par un groupe trifluorométhyle ou un groupe phényle substitué à la fois par 1 à 3 atomes d'halogène et par 1 ou 2 groupements méthyle ;
- $R_2$ représente l'hydrogène ou un groupe alkyle en $C_1$-$C_4$
- $R_3$ représente :
- un groupe

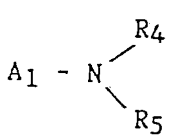

dans lequel

dans lequel

$A_1$ désigne un groupe alkyle droit en $C_3$-$C_4$

$R_4$ et $R_5$ considérés indépendamment représentent l'hydrogène, un groupe alkyle en $C_1$-$C_4$, ou encore $R_4$ et $R_5$ considérés avec l'atome d'azote auquel ils sont liés constituent un hétérocycle à 5 ou 6 chaînons contenant éventuellement un second hétéroatome et notamment l'un des cycles pyrrolidine, imidazole, pipéridine, morpholine, pipérazine ou N-alkylpipérazine ;

- un groupe

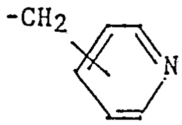

où le groupe méthylène substitue le cycle pyridine en position 3 ou 4 ;
- un groupe

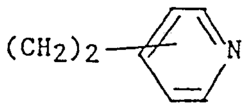

où le groupe éthylène substitue le cycle pyridinique en position 2 ou 3 ;
- un groupe

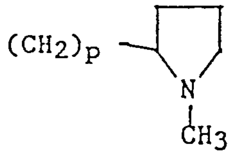

avec p = 2 ou 3 ;
- un groupe

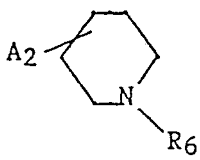

où $R_6$ désigne un groupe méthyle ou éthyle et $A_2$ désigne un groupe $(CH_2)_m$ où m = 1 ou 2, le groupe $A_2$ étant en position 3 ou 4 du cycle pipéridine lorsque m est 1, et en position 2 ou 3 de celui-ci lorsque m est 2.
- un groupe

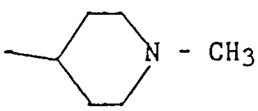

ou encore le substituant

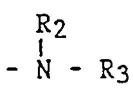

représente un groupe

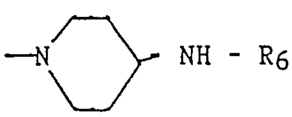

où $R_6$ est comme indiqué ci-dessus ainsi que leurs sels d'addition avec les acides minéraux ou organiques.

2. Composés selon la revendication 1 où $R_3$ représente :
- un groupe

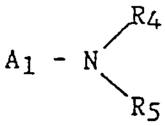

dans lequel
$A_1$ désigne un groupe alkyle droit en $C_3$ - $C_4$
$R_4$ et $R_5$ considérés indépendamment représentent l'hydrogène, un groupe alkyle en $C_1$-$C_4$, ou encore $R_4$ et $R_5$ considérés avec l'atome d'azote auquel ils sont liés constituent un hétérocycle à 5 ou 6 chaînons contenant éventuellement un second hétéroatome et notamment l'un des cycles pyrrolidine, imidazole, pipéridine, morpholine, pipérazine ou N-alkylpipérazine.

3. Composés selon la revendication 1 où $R_3$ représente :
- un groupe

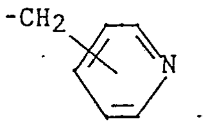

où le groupe méthylène substitue le cycle pyridine en position 3 ou 4 ;

4. Composés selon la revendication 1 où $R_3$ représente :
- un groupe

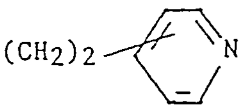

où le groupe éthylène substitue le cycle pyridinique en position 2 ou 3 ;

5. Composés selon la revendication 1 où $R_3$ représente :
- un groupe

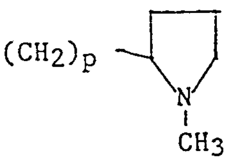

avec $p = 2$ ou 3.

6. Composés selon la revendication 1 où $R_3$ représente
- un groupe

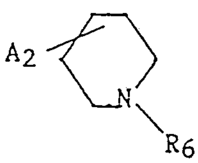

où $A_2$ désigne un groupe $(CH_2)m$ avec $m = 1$ ou 2, et
$R_6$ désigne un groupe méthyle ou éthyle.

7. Composés selon la revendication 1 où $R_3$ représente :
- un groupe

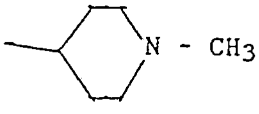

8. Composés selon la revendication 1 où le substituant

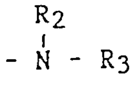

représente un groupe :

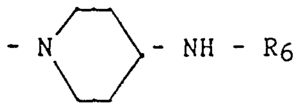

où $R_6$ désigne un groupe méthyle ou éthyle

9. Procédé d'obtention des composés de formule (I) selon la revendication 1, caractérisé en ce que l'on traite un halogéno-2 thiadiazole-1,3,4 correspondant de formule :

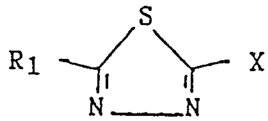

(II)

dans laquelle X représente le chlore ou le brome et $R_1$ est tel que défini dans la revendication 1, avec un composé de formule :

- HN $R'_2$ $R'_3$ (III)

dans laquelle $R'_2$ et $R'_3$ ont les mêmes significations que $R_2$ et $R_3$ définis ci-dessus ou représentent l'un ou l'autre un groupe protecteur lorsque $R_2$ et $R_3$ sont tous les deux l'hydrogène et on transforme, éventuellement, le produit ainsi obtenu dans un de ses sels pharmaceutiquement acceptables.

10. Procédé selon la revendication 9, caractérisé en ce que la réaction de substitution (II) + (III) est réalisée en présence, soit d'un réactif qui fixe l'hydracide XH formé au cours de la réaction, soit d'un exès d'amine (III), à température comprise entre 40° C et 130° C, dans un solvant polaire ou apolaire.

11. Procédé d'obtention des composés de formule (I) selon la revendication 1, dans laquelle $R_1$ représente le groupe hydroxyphényle ou polyhydroxyphényle, caractérisé en ce qu'il consiste à soumettre à une déméthylation les composés de formule (I) dans lesquels $R_1$ représente un groupe méthoxyphényle ou polyméthoxyphényle obtenus par le procédé selon l'une des revendications 9 ou 10.

12. Procédé d'obtention des composés de formule (I), selon la revendication 1, dans laquelle $R_3$ représente le groupe $A_1$-$NR_4R_5$ dans lequel $A_1$ est le groupe tétraméthylène, caractérisé en ce qu'il consiste à soumettre à une hydrogénation catalytique le dérivé acétylénique du thiadiazole de formule V:

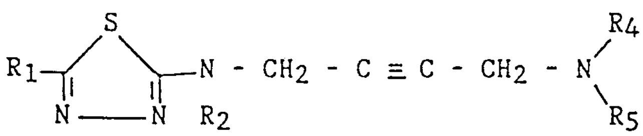

(V)

dans laquelle $R_1$, $R_2$, $R_4$ et $R_5$ sont tels que définis dans la revendication 1.

13. Utilisation des dérivés selon l'une quelconque des revendications 1 à 8, pour la préparation de médicaments agonistes du système cholinergique muscarinique.

Office européen des brevets

# RAPPORT DE RECHERCHE EUROPEENNE

Numero de la demande

EP 89 40 2322

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 183 577 (SANOFI)<br>* En entier *<br>----- | 1,9,13 | C 07 D 285/135<br>C 07 D 417/12<br>A 61 K 31/41<br>A 61 K 31/435 |
| | | | **DOMAINES TECHNIQUES RECHERCHES (Int. Cl.5)** |
| | | | C 07 D 285/00<br>C 07 D 417/00 |

Le présent rapport a été établi pour toutes les revendications

| Lieu de la recherche | Date d'achèvement de la recherche | Examinateur |
|---|---|---|
| LA HAYE | 13-11-1989 | ALLARD M.S. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant